# EUROPEAN PATENT APPLICATION

(11) **EP 4 814 808 A1**
(43) Date of publication of application: **30.09.2026**
(21) Application number: 26159834.6
(22) Date of filing: 20.02.2026
(51) Int. Cl.: G06N 10/60, G06N 3/0455

(54) **METHOD AND SYSTEM FOR COMPUTATION OF MOLECULAR PROPERTIES VIA QUANTUM AUTOENCODERS**

(30) Priority: 24.03.2025 IN 202521027393
(71) Applicant: Tata Consultancy Services Limited, Maharashtra (IN)
(72) Inventor: KHANDELWAL, Ankit, 560066 Bengaluru (IN); RAJAN, Nirmal Mammavalappil, 560066 Bengaluru (IN); CHANDRA, Mariswamy Girish, 560066 Bengaluru (IN)
(74) Representative: Goddar, Heinz J.

(57) **Abstract**

A method and system for computation of molecular properties via quantum autoencoders is disclosed. The disclosure provides quantum machine learning-based method to compress molecular Hamiltonians and efficiently relevant property of a molecule given at a geometry, thereby significantly reducing resource requirements. The method disclosed leverages a quantum autoencoder which is trained using ground states corresponding to molecular geometries of the molecule and a classical optimizer. An n-quantum bit (qubit) transformed property operator of an n- qubit property operator is determined using the trained quantum autoencoder. Further, n-qubit transformed property operator is compressed by performing a series of Pauli term processing to obtain a m-qubit compressed property operator. Further, a relevant molecular property at the geometry is determined using a variational quantum eigen solver (VQE) or using compressed ground state. The present disclosure is used to determine any property of the molecule at a given geometry using a reduced resource requirement.

## Description

### CROSS-REFERENCE TO RELATED APPLICATIONS AND PRIORITY

The present application claims priority to Indian application no. 202521027393, filed on March 24, 2025.

### TECHNICAL FIELD

The disclosure herein generally relates to quantum computing for quantum chemistry, and, more particularly, to a method and system for computation of molecular properties via quantum autoencoders.

### BACKGROUND

Quantum computing has the potential to revolutionize many fields, and one area where its impact is especially promising is quantum chemistry. Quantum chemistry involves the study of molecules and atoms at the quantum level, using principles from quantum mechanics to understand their behavior and interactions. The challenge, however, is that accurate simulation of many of these systems are computationally intractable with classical computers, especially as molecules grow larger and more complex.

Quantum chemistry, when simulated on classical computers, presents several significant challenges as discussed henceforth. The first and foremost problem is that the computational resources required to simulate a quantum system scale exponentially with the number of particles such as electrons and atoms. Classical computers quickly reach their limits for systems with large number of particles. Also, when handling large molecules, methods like Hartree-Fock or Density Functional Theory (DFT) are usually used to predict the molecular properties, which rely on many approximations to make quantum chemistry calculations tractable. These methods may not always yield highly accurate results, especially for a highly correlated system where more sophisticated methods would be needed. The amount of data generated by quantum chemistry simulations can be vast, especially when calculating large molecules or performing high-level calculations. Storing and manipulating this data efficiently on classical computers is a significant challenge. This requires specialized hardware or parallel computing architecture.

Quantum computers, which use quantum bits (qubits) instead of classical bits, can theoretically perform certain types of calculations exponentially faster than classical computers. This can significantly improve the modeling of quantum systems in chemistry, providing deeper insights into molecular structure, reaction mechanisms, and molecular properties.

Quantum computing offers several advantages in the field of quantum chemistry, primarily due to its ability to model complex quantum systems more efficiently than classical computers. The advantages of quantum computing include accurate molecular simulations, speed and efficiency, resource reduction in molecular property calculation and the like. These advantages hold significant potential in the practical application of quantum computing in quantum chemistry.

Already existing quantum computing methods like Parity mapping and Bravyi-Kitaev (BK) mapping techniques when combined with molecular symmetries, like z2 symmetries enable the removal of a few qubits from the property operator. These methods are analytical methods. However, these methods require identification of certain symmetries in the property operator to remove the qubits. It is not always possible (or it is not easy) to identify such symmetries for a complex chemical system and remove qubits. Sometimes, symmetries might not even exist, and property operator compression may not be possible.

### SUMMARY

Embodiments of the present disclosure present technological improvements as solutions to one or more of the above-mentioned technical problems recognized by the inventors in conventional systems. For example, in one embodiment, a method for computation of molecular properties via quantum autoencoders is provided. The method includes encoding, via the one or more classical hardware processors and the plurality of QPUs, an n-qubit property operator corresponding to a property of a molecule at a geometry received from a user via a user interface, using a quantum autoencoder trained using a training dataset to an n-qubit transformed property operator. Further the method includes compressing, via the one or more classical hardware processors and the plurality of QPUs, the n-qubit transformed property operator into a m-qubit compressed property operator by processing a set of Pauli terms comprised in the n-qubit transformed property operator. Here m is less than n. Finally the method includes, determining, via the one or more classical hardware processors and the plurality of QPUs, a relevant property of the molecule at the geometry based on the m-qubit compressed property operator, and at least one of (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule.

In another aspect, a system for computation of molecular properties via quantum autoencoders is provided. The system includes one or more classical hardware processors communicably coupled to a plurality of Quantum Processor Units (QPUs) via interfaces, wherein the one or more classical hardware processors comprises at least one memory storing programmed instructions; one or more Input/Output (I/O) interfaces; and one or more hardware processors operatively coupled to the at least one memory, wherein the one or more classical hardware processors and the plurality of QPUs are configured by the programmed instructions to encode, via the one or more classical hardware processors and the plurality of QPUs, an n-qubit property operator corresponding to a property of a molecule at a geometry received from a user via a user interface, using a quantum autoencoder trained using a training dataset to an n-qubit transformed property operator. Further, via the one or more classical hardware processors and the plurality of QPUs, the n-qubit transformed property operator is compressed into a m-qubit compressed property operator by processing a set of Pauli terms comprised in the n-qubit transformed property operator. Here m is less than n. Finally, via the one or more classical hardware processors and the plurality of QPUs, a relevant property of the molecule at the geometry is determined based on the m-qubit compressed property operator, and at least one of (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule.

The quantum autoencoder is trained using the training data set corresponding to a set of geometries associated with the molecule and a classical optimizer, wherein the training dataset is a set of ground states for the set of geometries.

The n-qubit transformed property operator is compressed into the m-qubit compressed property operator by performing steps as mentioned henceforth. First, via the one or more classical hardware processors and the plurality of QPUs, the n-qubit transformed property operator is represented into a sum of the set of Pauli terms. A Pauli term amongst the set of Pauli terms is represented using a coefficient and a Pauli string with a first set of qubits, wherein the Pauli string is formed from a set of Pauli operators. Then via the one or more classical hardware processors and the plurality of QPUs, a subset of Pauli terms amongst the set of Pauli terms having a subset of Pauli operators amongst the set of Pauli operators is removed from a first subset of qubits amongst the first set of qubits based on a set of predefined equations to receive a first group of Pauli terms. Finally, via the one or more classical hardware processors and the plurality of QPUs, a set of similar Pauli terms in the first group of Pauli terms is combined to obtain a second group of Pauli terms representing the m-qubit compressed property operator.

The m-qubit compressed ground state is determined using at least one of (i) the quantum autoencoder based on a n-qubit ground state received via one of (a) the one or more classical hardware processors, or (b) the plurality of QPUs, or (ii) the m-qubit compressed property operator, the initial state and the classical optimizer using the variational quantum eigen solver (VQE), if the n-qubit property operator is the Hamiltonian operator.

In yet another aspect, a computer program product including a non-transitory computer-readable medium having embodied therein a computer program for computation of molecular properties via quantum autoencoders is provided. The computer readable program, when executed on a system comprising one or more classical hardware processors communicably coupled to a plurality of Quantum Processor Units (QPUs) via interfaces, causes the computing device to encode, via the one or more classical hardware processors and the plurality of QPUs, an n-qubit property operator corresponding to a property of a molecule at a geometry received from a user via a user interface, using a quantum autoencoder trained using a training dataset to an n-qubit transformed property operator. Further, the computer readable program, when executed on the system comprising one or more classical hardware processors communicably coupled to a plurality of Quantum Processor Units (QPUs) via interfaces, causes the computing device to by the one or more classical hardware processors and the plurality of QPUs to compress the n-qubit transformed property operator into a m-qubit compressed property operator by processing a set of Pauli terms comprised in the n-qubit transformed property operator. Here m is less than n. Finally, the computer readable program, when executed on the system comprising one or more classical hardware processors communicably coupled to a plurality of Quantum Processor Units (QPUs) via interfaces, causes the computing device to by the one or more classical hardware processors and the plurality of QPUs to determine a relevant property of the molecule at the geometry based on the m-qubit compressed property operator, and at least one of (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is a functional block diagram of a system for computation of molecular properties via quantum autoencoders according to some embodiments of the present disclosure.
FIG.2 is an exemplary flow diagram illustrating a method for computation of molecular properties via quantum autoencoders according to some embodiments of the present disclosure.
FIG. 3 illustrates an example implementation for computing ground state energy of the molecule via quantum autoencoder according to some embodiments of the present disclosure.
FIG.4A through FIG. 4C illustrates potential energy surfaces for molecules H₂, LiH, BeH₂ computed using prior art methods and the method disclosed according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Quantum autoencoder is a type of quantum neural network that aims to compress quantum information in a manner like classical autoencoders but in a quantum setting. The quantum autoencoder reduces the dimensionality of quantum data while preserving the essential quantum properties. The quantum autoencoder performs a similar function to that of a classical autoencoder but operates on quantum states instead of classical data. It maps quantum states to a lower dimensional quantum subspace and then decodes them back to approximate the original state.

Embodiments of the present disclosure provide a method for computing molecular properties using quantum autoencoders. A quantum autoencoder is trained using ground states corresponding to molecular geometries of a molecule and a classical optimizer. This trained quantum autoencoder is used for determining a n-quantum bit (qubit) transformed property operator of an n- qubit property operator. Further this n-qubit transformed property operator is compressed to obtain a m-qubit compressed property operator, where m is less than n. This compression is performed by a series of Pauli term processing. Further, a relevant property of the molecule at the geometry is determined using a variational quantum eigen solver (VQE) or using a m-qubit compressed ground state. The present disclosure is used to determine any molecular property of the molecule at a given geometry using a reduced resource requirement, thereby increasing the time efficiency and power efficiency. The disclosed method for operator compression has an advantage that the user does not need to have chemical intuition or knowledge of the molecular symmetries to remove qubits and compress the property operator. Also, it's possible to go beyond the compression possible by the analytical methods already available in the art with the method disclosed.

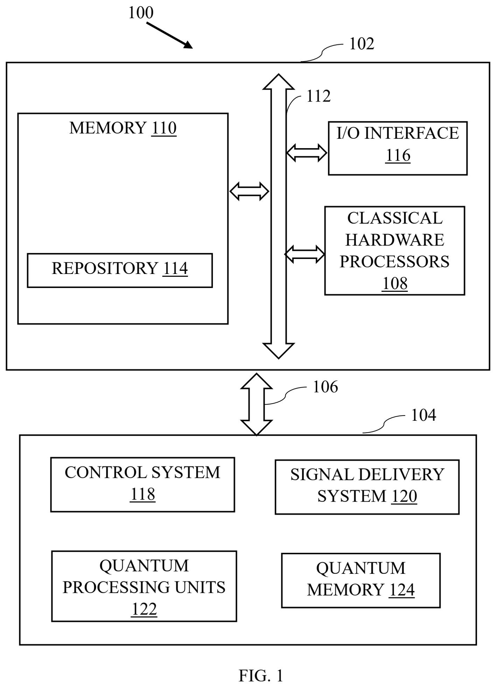

Referring now to the drawings, and more particularly to FIG. 1 through FIG. 4C, where similar reference characters denote corresponding features consistently throughout the figures, there are shown preferred embodiments and these embodiments are described in the context of the following exemplary system and/or method.

FIG. 1 is a functional block diagram of a system for computation of molecular properties via quantum autoencoders according to some embodiments of the present disclosure. The system 100 includes a classical computing system 102, a quantum computing system 104 and a communication interface 106.

The classical computing system 102 comprises classical hardware processors 108, at least one memory such as a memory 110, an I/O interface 116. The classical hardware processors 108, the memory 110, and the Input /Output (I/O) interface 116 may be coupled by a system bus such as a system bus 112 or a similar mechanism. In an embodiment, the classical hardware processors 108 can be one or more hardware processors. The classical hardware processors and the hardware processors is interchangeably used throughout the document. Similarly, the classical computing system is a normal computing system.

The I/O interface 116 may include a variety of software and hardware interfaces, for example, a web interface, a graphical user interface, and the like., for example, interfaces for peripheral device(s), such as a keyboard, a mouse, an external memory, a printer and the like. Further, the I/O interface 116 may enable the system 100 to communicate with other devices, such as web servers, and external databases. The I/O interface 116 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 116 may include one or more ports for connecting several computing systems with one another or to another server computer.

The one or more hardware processors 108 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 108 is configured to fetch and execute computer-readable instructions stored in the memory 110.

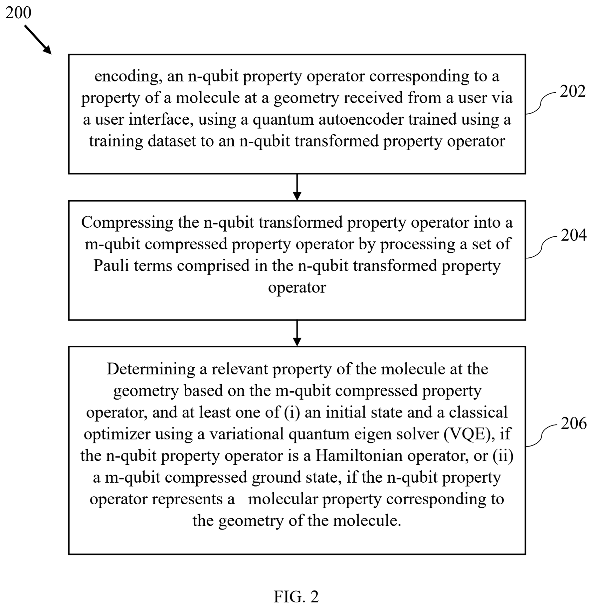

The memory 110 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 110 includes a data repository 114. The data repository (or repository) 114 may include a plurality of abstracted piece of code for refinement and data that is processed, received, or generated as a result of the execution of the method illustrated in FIG.2. Although the data repository 114 is shown internal to the system 100, it should be noted that, in alternate embodiments, the data repository 114 can also be implemented external to the system 100, where the data repository 114 may be stored within a database (repository 114) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

Referring now to quantum computing system 104 shown in FIG. 1 includes a control system 118, a signal delivery system 120, a plurality of Quantum Processing Units (QPUs) 122 and a quantum memory 124. The plurality of QPUs is unentangled and hence alternatively called the plurality of unentangled QPUs. The quantum computing system 104 may include additional or different features, and the components of a quantum computing system may operate as described with respect to FIG. 1 or in another manner.

The example quantum computing system 104 shown in FIG. 1 can perform quantum computational tasks (such as, for example, quantum simulations or other quantum computational tasks) by executing quantum algorithms. In some implementations, the quantum computing system 104 can perform quantum computation by storing and manipulating information within individual quantum states of a composite quantum system. For example, Qubits (i.e., Quantum bits) can be stored in and represented by an effective two-level sub-manifold of a quantum coherent physical system in the plurality of QPUs 122. In an embodiment, the quantum computing system 104 can operate using gate-based models for quantum computing. For example, the Qubits can be initialized in an initial state, and a quantum logic circuit comprised of a series of quantum logic gates can be applied to transform the qubits and extract measurements representing the output of the quantum computation. The example QPUs 122 shown in FIG.1 may be implemented, for example, as a superconducting quantum integrated circuit that includes Qubit devices. The Qubit devices may be used to store and process quantum information, for example, by operating as ancilla Qubits, data Qubits or other types of Qubits in a quantum algorithm. Coupler devices in the superconducting quantum integrated circuit may be used to perform quantum logic operations on single qubits or conditional quantum logic operations on multiple qubits. In some instances, the conditional quantum logic can be performed in a manner that allows large-scale entanglement within the QPUs 122. Control signals may be delivered to the superconducting quantum integrated circuit, for example, to manipulate the quantum states of individual Qubits and the joint states of multiple Qubits. In some instances, information can be read from the superconducting quantum integrated circuit by measuring the quantum states of the qubit devices. The QPUs 122 may be implemented using another type of physical system.

The example QPUs 122, and in some cases all or part of the signal delivery system 120, can be maintained in a controlled cryogenic environment. The environment can be provided, for example, by shielding equipment, cryogenic equipment, and other types of environmental control systems. In some examples, the components in the QPUs 122 operate in a cryogenic temperature regime and are subject to very low electromagnetic and thermal noise. For example, magnetic shielding can be used to shield the system components from stray magnetic fields, optical shielding can be used to shield the system components from optical noise, thermal shielding and cryogenic equipment can be used to maintain the system components at controlled temperature, etc.

In the example shown in FIG. 1, the signal delivery system 120 provides communication between the control system 118 and the QPUs 122. For example, the signal delivery system 120 can receive control signals from the control system 118 and deliver the control signals to the QPUs 122. In some instances, the signal delivery system 120 performs preprocessing, signal conditioning, or other operations to the control signals before delivering them to the QPUs 122. In an embodiment, the signal delivery system 120 includes connectors or other hardware elements that transfer signals between the QPUs 122 and the control system 118. For example, the connection hardware can include signal lines, signal processing hardware, filters, feedthrough devices (e.g., light-tight feedthroughs, etc.), and other types of components. In some implementations, the connection hardware can span multiple different temperature and noise regimes. For example, the connection hardware can include a series of temperature stages that decrease between a higher temperature regime (e.g., at the control system 118) and a lower temperature regime (e.g., at the QPUs 122).

In the example quantum computer system 104 shown in FIG. 1, the control system 118 controls operation of the QPUs 122. The example control system 118 may include data processors, signal generators, interface components and other types of systems or subsystems. Components of the example control system 118 may operate in a room temperature regime, an intermediate temperature regime, or both. For example, the control system 118 can be configured to operate at much higher temperatures and be subject to much higher levels of noise than are present in the environment of the QPUs 122. In some embodiments, the control system 118 includes a classical computing system that executes software to compile instructions for the QPUs 122. For example, the control system 118 may decompose a quantum logic circuit or quantum computing program into discrete control operations or sets of control operations that can be executed by the hardware in the QPUs 122. In some examples, the control system 118 applies a quantum logic circuit by generating signals that cause the Qubit devices and other devices in the QPUs 122 to execute operations. For instance, the operations may correspond to single-Qubit gates, two-Qubit gates, Qubit measurements, etc. The control system 118 can generate control signals that are communicated to the QPUs 122 by the signal delivery system 120, and the devices in the QPUs 122 can execute the operations in response to the control signals.

In some other embodiments, the control system 118 includes one or more classical computers or classical computing components that produce a control sequence, for instance, based on a quantum computer program to be executed. For example, a classical processor may convert a quantum computer program to an instruction set for the native gate set or architecture of the QPUs 122. In some cases, the control system 118 includes a microwave signal source (e.g., an arbitrary waveform generator), a bias signal source (e.g., a direct current source) and other components that generate control signals to be delivered to the QPUs 122. The control signals may be generated based on a control sequence provided, for instance, by a classical processor in the control system 118. The example control system 118 may include conversion hardware that digitizes response signals received from the QPUs 122. The digitized response signals may be provided, for example, to a classical processor in the control system 118.

In some embodiments, the quantum computer system 104 includes multiple quantum information processors that operate as respective quantum processor units (QPU). In some cases, each QPU can operate independent of the others. For instance, the quantum computer system 104 may be configured to operate according to a distributed quantum computation model, or the quantum computer system 104 may utilize multiple QPUs in another manner. In some implementations, the quantum computer system 104 includes multiple control systems, and each QPU may be controlled by a dedicated control system. In some implementations, a single control system can control multiple QPUs; for instance, the control system 118 may include multiple domains that each control a respective QPU. In some instances, the quantum computing system 104 uses multiple QPUs to execute multiple unentangled quantum computations (e.g., multiple Variational Quantum Eigen solver (VQE)) that collectively simulate a single quantum mechanical system.

In an embodiment, the quantum memory 124 is a quantum-mechanical version of classical computer memory. The classical computer memory stores information such as binary states and the quantum memory 124 stores a quantum state for later retrieval. These states hold useful computational information known as Qubits. In an embodiment, the communication interface 106 which connects the classical computing system 102 and the quantum computing system 104 is a high speed digital interface.

FIG.2 is an exemplary flow diagram illustrating a method for computation of molecular properties via quantum autoencoders according to some embodiments of the present disclosure. In an embodiment, the system 100 includes one or more data storage devices or the memory 110 operatively coupled to the one or more hardware processor(s) 108 and is configured to store instructions for execution of steps of the method 200 by the one or more hardware processors 108. The steps of the method 200 of the present disclosure will now be explained with reference to the components or blocks of the system 100 as depicted in FIG. 1. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIG. 2, at step 202 of the method 200, one or more classical hardware processors 108 and the plurality of QPUs 122 are configured to encode an n-qubit property operator corresponding to a property of a molecule at a geometry, which is received from a user via a user interface of the system 100. The n-qubit property operator is encoded using a quantum autoencoder trained using a training dataset to obtain an n-qubit transformed property operator. The quantum autoencoder is trained using the training data set corresponding to a set of geometries associated with the molecule along with a classical optimizer. The training dataset is a set of ground states for the set of geometries associated with the molecule. The set of ground states are represented as $\left\{\left|{Ψ}_{gs}^{d}\right〉\right\}$, where *d* represents some geometry, *gs* represents ground state. The quantum autoencoder (*qa*) is trained and is represented as *U_{A}*(*̅θ̅*̅), where *̅θ̅*̅ are the trainable parameters. The quantum autoencoder (*qa*) is trained by sending the k trash qubits (k=input qubits (n) - output qubits (m)) to a predefined state |α〉, for example |α〉 is equal to the zero state, i.e., |0〉^{⊗*k*}. Once trained the action of an ideal fully trained quantum autoencoder on an n-qubit ground state is given by ${U}_{A} \left(\vec{θ}\right) \left|{Ψ}_{gs}^{d}\right〉 = \left|α\right〉 \left|{Φ}_{c}^{d}\right〉 = \left|π\right〉$ where the subscript 'c' is used to represent the compressed nature and $\left|{Φ}_{c}^{d}\right〉$ is the compressed ground state.

Given the n-qubit property operator *O^{d}* that represents some property P of the molecule at the geometry d , its action on the n-qubit ground state is given by $\left〈{Ψ}_{ds}^{d}\right| {O}^{d} \left|{Ψ}_{ds}^{d}\right〉 = {P}_{gs}^{d}$ where ${P}_{gs}^{d}$ is the value of the property P for the ground state at the geometry d. The n-qubit property operator is encoded to transform into the n-qubit transformed property operator. This is represented as, ${O}_{t}^{d} = {U}_{A} \left(\vec{θ}\right) {O}^{d} {U}_{A} {\left(\vec{θ}\right)}^{†}$ The subscript 't' represents the transformed nature. The action of the transformed property operator is defined as: $\left〈π\right| {O}_{t}^{d} \left|π\right〉 = {P}_{gs}^{d}$ Expanding this, the equation below is obtained, $\left〈{Φ}_{c}^{d}\left|\left〈α\left|{O}_{t}^{d}\right|α\right〉\right|{Φ}_{c}^{d}\right〉 = {P}_{gs}^{d}$

Further, at step 204 of the method 200, the one or more classical hardware processors 108 and the plurality of QPUs 122 are configured to compress the n-qubit transformed property operator into a m-qubit compressed property operator by processing a set of Pauli terms comprised in the n-qubit transformed property operator. Here as mentioned before m is less than n. The processing of the set of Pauli terms is explained henceforth. Any property operator can be decomposed into a sum of the set of Pauli terms, *O* = *Σᵢ cᵢPᵢ* , where *cᵢ* and *Pᵢ* are the coefficients and the corresponding n-qubit (first set of qubits) Pauli strings, respectively. Each Pauli string *Pᵢ* is in the form of ${p}_{i}^{1} ⊗ {p}_{i}^{2} ⊗ {p}_{i}^{3} … . ⊗ {p}_{i}^{n}$ where *pᵢ* can be any of the set of Pauli operators (*I, X, Y, Z*). Then a subset of Pauli terms from the set of Pauli terms which is having a subset of Pauli operators is removed from a first subset of qubits amongst the first set of qubits (n). This removal is based on a set of predefined equations and after removal, a first group of Pauli terms is obtained. The set of predefined equations are $\left〈α\left|{⊗}_{i = 1}^{k}{p}_{i}\right|α\right〉 =$ *β*. Those Pauli terms that have *β* = 0 are removed from 0, as these terms will not add to the expectation value. For the remaining Pauli terms (with non-zero *β*), the k trash qubits are removed as their contribution to the expectation value is now known to be *β*. Further a set of similar Pauli terms in the first group of Pauli terms is combined to obtain a second group of Pauli terms representing the m-qubit compressed property operator. Thus, output after this processing is the m-qubit compressed property operator and is represented as $\left〈{Φ}_{c}^{d}\right| \left〈α\left|{O}_{t}^{d}\right|α\right〉 \left|{Φ}_{c}^{d}\right〉 = {P}_{gs}^{d} = \left〈{Φ}_{c}^{d}\right| {O}_{c}^{d} \left|{Φ}_{c}^{d}\right〉$ where ${O}_{c}^{d}$ is the m-qubit compressed property operator.

At step 206 of the method 200, the one or more classical hardware processors 108 and the plurality of QPUs 122 are configured to determine a relevant property of the molecule at the geometry based on the m-qubit compressed property operator, and at least one of the following, (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule.

If the n-qubit property operator is Hamiltonian operator, the relevant property is determined using the m-qubit compressed property operator and the initial state and the classical optimizer using the VQE. If the n-qubit property operator is Hamiltonian, *O^{d} = H^{d},* $\left〈{Ψ}_{gs}^{d}\right| {H}^{d} \left|{Ψ}_{gs}^{d}\right〉 = {E}_{gs}^{d}$, and ${O}_{c}^{d} = {H}_{c}^{d}$, where ${E}_{gs}^{d}$ is the corresponding ground state energy for the ground state $\left|{Ψ}_{gs}^{d}\right〉$. For the given geometry d for inference, VQE is performed on the latent space by using the compressed Hamiltonian as the operator and some initial state (here for example zero state is considered) for the given geometry to get the corresponding ground state energy. The m-qubit initial state is represented as |0〉^{⊗*m*}. Then the appropriate VQE ansatz represented as *V*(*̅θ̅*̅) is chosen. An "ansatz" is a parameterized quantum state or trial wavefunction used as a starting point for approximations or optimizations, serving as a guess for solving a problem like finding the ground state of a quantum system. Perform the VQE to minimize the expectation value to get the ground state energy, the property of the molecule. This is represented as, ${\left〈0\right|}^{⊗ m} V {\left(\vec{θ}\right)}^{†} {H}_{c}^{d} \left(\vec{θ}\right) V \left(\vec{θ}\right) {\left|0\right〉}^{⊗ m} = {E}_{gs}^{d}$ $V \left(\vec{θ}\right) {\left|0\right〉}^{⊗ m} = \left|{Φ}_{gs}^{d}\right〉$ Thus, the m-qubit compressed ground state, and the ground state energy is determined.

If the n-qubit property operator is not the Hamiltonian, and any other property operator, such as dipole moment and the like, then the m-qubit compressed ground state with the m-qubit compressed property operator is used to get the corresponding property value. This is represented as, $\left〈{Φ}_{c}^{d}\right| {O}_{c}^{d} \left|{Φ}_{c}^{d}\right〉 = {P}_{gs}^{d}$ The m-qubit compressed ground state used in the above equation can be determined using two ways. The first way is by using the quantum autoencoder and an n-qubit ground state. Given $\left|{Ψ}_{gs}^{d}\right〉$, the quantum autoencoder is applied on this, represented as ${U}_{A} \left(\vec{θ}\right) \left|{Ψ}_{gs}^{d}\right〉 = \left|α\right〉 \left|{Φ}_{gs}^{d}\right〉$. This n-qubit ground state is received via the one or more classical hardware processors, or from the plurality of QPUs. The second way is when the n-qubit property operator is the Hamiltonian operator. This is done by using the VQE based on the m-qubit compressed property operator, the initial state and the classical optimizer.

### EXPERIMENTAL RESULTS:

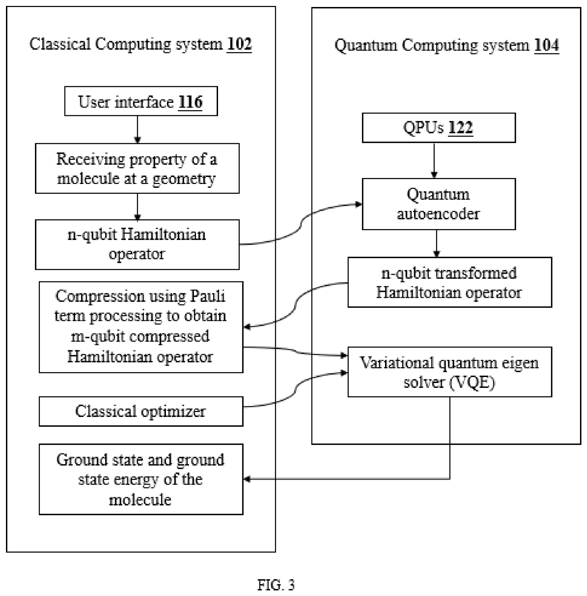

PennyLane and Qiskit were used to simulate the quantum algorithm and demonstrate it on four-qubit H₂, six-qubit LiH, and eight-qubit BeH₂ molecules which are trained using 5, 10, and 10 data points, respectively. Though PennyLane and Qiskit are used only for experimental purposes, any suitable framework can be used to achieve the desired results. As the training data is small, the initial cost of training can be set off against the savings from running the compressed VQE. FIG. 3 illustrates an example implementation for computing ground state energy of the molecule via quantum autoencoder according to some embodiments of the present disclosure. It is to be noted that steps 202 through 206 depicted in FIG. 2 can be implemented via the classical computer system 102 or the quantum computing system 104. Referring FIG.3, an example embodiment for explaining the steps 202 through 206, if the property operator is Hamiltonian operator is provided and the explanation henceforth. The property of the molecule at the geometry is received via the user interface in the classical computing system 102. Further the n-qubit Hamiltonian operator is encoded using the quantum autoencoder via the QPUs 122 in the quantum computing system 104 to obtain the n-qubit transformed Hamiltonian operator. This n-qubit transformed Hamiltonian operator is further compressed using the Pauli term processing in the classical computing system 102 via the one or more hardware processors to obtain the m-qubit compressed Hamiltonian operator. Finally, the ground state and ground state energy of the molecule at the geometry is determined as the relevant property by the VQE using the m-qubit compressed Hamiltonian operator and the classical optimizer. This is done in the quantum computing system via the QPUs.

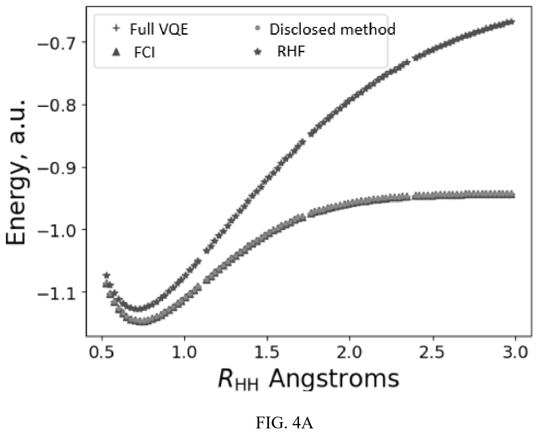

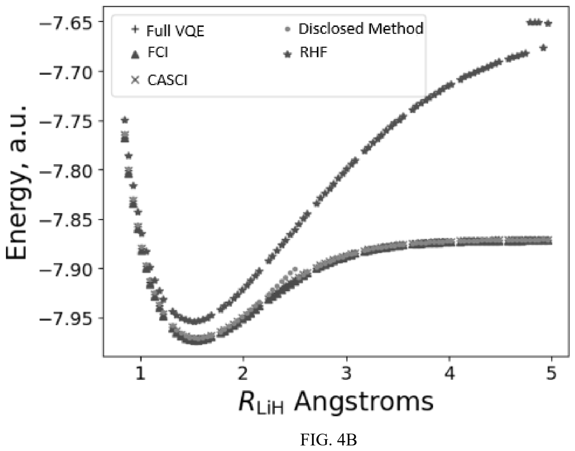

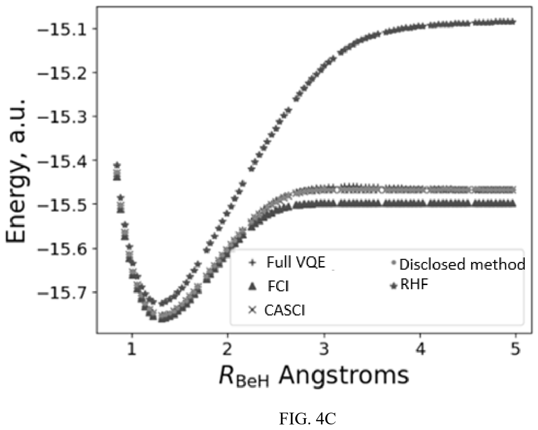

FIG.4A, FIG. 4B and FIG. 4C illustrate the potential energy surfaces for molecules H₂, LiH, BeH₂ computed using prior art methods and the method disclosed according to some embodiments of the present disclosure. Table 1 shows the testing metrics for these different molecules considered in the method disclosed. The method disclosed can cross the chemical accuracy threshold of 1.6 mHa for all molecules tested. Table 1 shows computed metrics for the testing data for the different molecules and the achieved compression n→m. MAE is the mean absolute error between the ground state energy calculated from the method disclosed and Full configuration interaction (FCI) for H₂ and Complete-active-space configuration interaction (CASCI) for LiH and BeH₂. NPE is the non-parallelity error defined as the difference between the maximum and minimum absolute energy errors. Fidelity is calculated between the output decoded state from the quantum autoencoder and the original input state. Hartree Fock (HF) Von Neumann entanglement entropy (VNEE) is the VNEE between the trash and latent space when the HF state is sent as input to the trained autoencoder.

**Table 1**

| **Molecule** | **Compression** | **MAE(Hartree)** | **NPE (Hartree)** | **Fidelity** | **HF VNEE** |
|---|---|---|---|---|---|
| H₂ | 4 → 1 | 2.29966 × 10⁻¹³ | 9.95648 × 10⁻¹³ | 1.000 | 2.241 × 10⁻¹² |
| LiH | 6 → 2 | 0.00129 | 0.01126 | 0.998 | 0.0081 |
| BeH₂ | 8 → 5 | 0.00036 | 0.00175 | 1.000 | 1.407 × 10⁻⁷ |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method for computation of molecular properties via quantum autoencoders (200), comprising:
encoding, via the one or more classical hardware processors and a plurality of Quantum Processor Units (QPUs), an n-qubit property operator corresponding to a property of a molecule at a geometry received from a user via a user interface to an n-qubit transformed property operator, using a quantum autoencoder (202);
compressing, via the one or more classical hardware processors and the plurality of QPUs, the n-qubit transformed property operator into a m-qubit compressed property operator by processing a set of Pauli terms comprised in the n-qubit transformed property operator, wherein m is less than n (204); and
determining, via the one or more classical hardware processors and the plurality of QPUs, a relevant property of the molecule at the geometry based on the m-qubit compressed property operator, and at least one of (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule (206).

2. The method as claimed in claim 1, wherein the quantum autoencoder is trained using a training data set corresponding to a set of geometries associated with the molecule and a classical optimizer, wherein the training dataset is a set of ground states for the set of geometries.

3. The method as claimed in claim 1, wherein the n-qubit transformed property operator is compressed into the m-qubit compressed property operator by,
representing, via the one or more classical hardware processors and the plurality of QPUs, the n-qubit transformed property operator into a sum of the set of Pauli terms, wherein a Pauli term amongst the set of Pauli terms is represented using a coefficient and a Pauli string with a first set of qubits, wherein the Pauli string is formed from a set of Pauli operators;
removing, via the one or more classical hardware processors and the plurality of QPUs, a subset of Pauli terms amongst the set of Pauli terms having a subset of Pauli operators amongst the set of Pauli operators from a first subset of qubits amongst the first set of qubits based on a set of predefined equations to receive a first group of Pauli terms; and
combining, via the one or more classical hardware processors and the plurality of QPUs, a set of similar Pauli terms in the first group of Pauli terms to obtain a second group of Pauli terms representing the m-qubit compressed property operator.

4. The method as claimed in claim 1, wherein the m-qubit compressed ground state is determined using at least one of (i) the quantum autoencoder based on a n-qubit ground state received via one of (a) the one or more classical hardware processors, or (b) the plurality of QPUs, or (ii) the m-qubit compressed property operator, the initial state and the classical optimizer using the variational quantum eigen solver (VQE), if the n-qubit property operator is the Hamiltonian operator.

5. A system (100), comprising:
one or more classical hardware processors (108) and a plurality of Quantum Processor Units (QPUs) (122), wherein the one or more classical hardware processors (108) are communicably coupled to the plurality of QPUs (122) by one or more communication interfaces (106), wherein the one or more classical hardware processors are operatively coupled to at least one memory (110) storing programmed instructions and one or more Input/Output (I/O) interfaces (116); and the plurality of quantum processor units (122) are operatively coupled to the at least one quantum memory (124), wherein the one or more hardware processors (108) and the plurality of QPUs (122) are configured by the programmed instructions to:
encode an n-qubit property operator corresponding to a property of a molecule at a geometry received from a user via a user interface, to an n-qubit transformed property operator using a quantum autoencoder;
compress the n-qubit transformed property operator into a m-qubit compressed property operator by processing a set of Pauli terms comprised in the n-qubit transformed property operator, wherein m is less than n; and
determine a relevant property of the molecule at the geometry based on the m-qubit compressed property operator, and at least one of (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule.

6. The system as claimed in claim 5, wherein the quantum autoencoder is trained using a training data set corresponding to a set of geometries associated with the molecule and a classical optimizer, wherein the training dataset is a set of ground states for the set of geometries.

7. The system as claimed in claim 5, wherein the n-qubit transformed property operator is compressed into the m-qubit compressed property operator by,
representing the n-qubit transformed property operator into a sum of the set of Pauli terms, wherein a Pauli term amongst the set of Pauli terms is represented using a coefficient and a Pauli string with a first set of qubits, wherein the Pauli string is formed from a set of Pauli operators;
removing a subset of Pauli terms amongst the set of Pauli terms having a subset of Pauli operators amongst the set of Pauli operators from a first subset of qubits amongst the first set of qubits based on a set of predefined equations to receive a first group of Pauli terms; and
combining a set of similar Pauli terms in the first group of Pauli terms to obtain a second group of Pauli terms representing the m-qubit compressed property operator.

8. The system as claimed in claim 5, wherein the m-qubit compressed ground state is determined using at least one of (i) the quantum autoencoder based on a n-qubit ground state received via one of (a) the one or more classical hardware processors, or (b) the plurality of QPUs, or (ii) the m-qubit compressed property operator, the initial state and the classical optimizer using the variational quantum eigen solver (VQE), if the n-qubit property operator is the Hamiltonian operator.

9. One or more non-transitory machine-readable information storage mediums comprising one or more instructions which when executed by one or more classical hardware processors and a plurality of Quantum Processor Units (QPUs) cause:
encoding an n-qubit property operator corresponding to a property of a molecule at a geometry received from a user via a user interface to an n-qubit transformed property operator, using a quantum autoencoder ;
compressing the n-qubit transformed property operator into a m-qubit compressed property operator by processing a set of Pauli terms comprised in the n-qubit transformed property operator, wherein m is less than n ; and
determining a relevant property of the molecule at the geometry based on the m-qubit compressed property operator, and at least one of (i) an initial state and a classical optimizer using a variational quantum eigen solver (VQE), if the n-qubit property operator is a Hamiltonian operator, or (ii) a m-qubit compressed ground state, if the n-qubit property operator represents a molecular property corresponding to the geometry of the molecule .

10. The one or more non-transitory machine-readable information storage mediums of claim 9, wherein the quantum autoencoder is trained using a training data set corresponding to a set of geometries associated with the molecule and a classical optimizer, wherein the training dataset is a set of ground states for the set of geometries.

11. The one or more non-transitory machine-readable information storage mediums of claim 9, wherein the n-qubit transformed property operator is compressed into the m-qubit compressed property operator by,
representing the n-qubit transformed property operator into a sum of the set of Pauli terms, wherein a Pauli term amongst the set of Pauli terms is represented using a coefficient and a Pauli string with a first set of qubits, wherein the Pauli string is formed from a set of Pauli operators;
removing a subset of Pauli terms amongst the set of Pauli terms having a subset of Pauli operators amongst the set of Pauli operators from a first subset of qubits amongst the first set of qubits based on a set of predefined equations to receive a first group of Pauli terms; and
combining a set of similar Pauli terms in the first group of Pauli terms to obtain a second group of Pauli terms representing the m-qubit compressed property operator.

12. The one or more non-transitory machine-readable information storage mediums of claim 9, wherein the m-qubit compressed ground state is determined using at least one of (i) the quantum autoencoder based on a n-qubit ground state received via one of (a) the one or more classical hardware processors, or (b) the plurality of QPUs, or (ii) the m-qubit compressed property operator, the initial state and the classical optimizer using the variational quantum eigen solver (VQE), if the n-qubit property operator is the Hamiltonian operator.
